# EUROPEAN PATENT SPECIFICATION

(11) **EP 0 229 729 B1**
(45) Date of publication and mention of the grant of the patent: **21.08.1996**
(21) Application number: 87300362.8
(22) Date of filing: 16.01.1987
(51) Int. Cl.: A61M 5/00, A61M 5/14

(54) **Implantable treatment reservoir**
Implantierbarer Medikamentenspeicher
Réservoir implantable pour médicament

(30) Priority: 17.01.1986 US 820714
(43) Date of publication of application: 22.07.1987
(73) Proprietor: STRATO/INFUSAID INC., Norwood, MA 02062 (US)
(72) Inventor: Fenton, Paul V., Jr., Marblehead Massachusetts 01945 (US); Young, Thomas M., North Andover Massachusetts 01845 (US)
(74) Representative: Harvey, David Gareth

(56) References cited:
- EP-A- 0 110 117
- EP-A- 0 119 596
- EP-A- 0 134 745
- EP-A- 0 183 351
- FR-A- 2 479 692
- US-A- 4 548 607

## Description

This invention relates to implantable assemblies comprising treatment material reservoirs for providing a treatment material, such as a drug in fluid form, directly to the vascular system of a mammal. A number of such assemblies are known, and may be broadly described as having a housing which defines a reservoir for holding the treatment material, and a catheter leading from the housing for interconnecting the reservoir with the vascular system. Existing implantable assemblies are adapted to be sutured into place within the body.

The existing assemblies known to the inventor are of two types. The first type has a catheter permanently affixed to the reservoir housing. Such permanent affixation makes the assembly awkward to implant. The second type has a catheter which fits over a male tube projecting from the housing, and is secured thereto by placing an external collar about the catheter. This assembly permits the housing to be sutured in position, and the catheter to be installed in a vein and sized before connection of the catheter to the housing. However, the connection of the catheter requires care and it relies solely on elastic gripping to remain attached. It is desirable to provide an implantable reservoir device which permits the direct and simple yet secure connection to a catheter after the suturing of the reservoir housing in the body and the placement and sizing of the catheter. EP-A 0119596 has the catheter secured in position by an annular protrusion on the male tube rather than by a collar.

The present invention is an implantable assembly as defined in claim 1. Preferred features of the assembly are recited in the sub-claims following claim 1.

Embodiments of the present invention will now be described, by way of example, with reference to the accompanying drawings, in which:
Figure 1 shows a cut-away pictorial view of an implantable assembly according to the invention;
Figure 2 shows a schematic section of the assembly of Figure 1 implanted beneath the skin of a mammal;
Figure 3A shows a perspective of a preferred female catheter connector;
Figure 3B shows a plane section through the preferred connector of Figure 3A;
Figure 4A shows a bottom view of a body portion of the assembly of Figure 1 having a mount formed on a housing thereof for accommodating the catheter connector of Figures 3A, 3B;
Figure 4B shows the side view seen from the catheter flow axis of the body portion shown in Figure 4A;
Figure 4C shows a vertical section of the body portion of Figures 4A, 4B, in a plane containing the flow axis;
Figure 5 shows a top view of the assembly of Figures 1 to 4 with the catheter secured in its twist-locked position;
Figure 6 shows a perspective view of an assembly comprising multiple reservoirs according to the invention; and
Figure 6A shows a detail of a preferred embodiment of a multiple reservoir assembly.

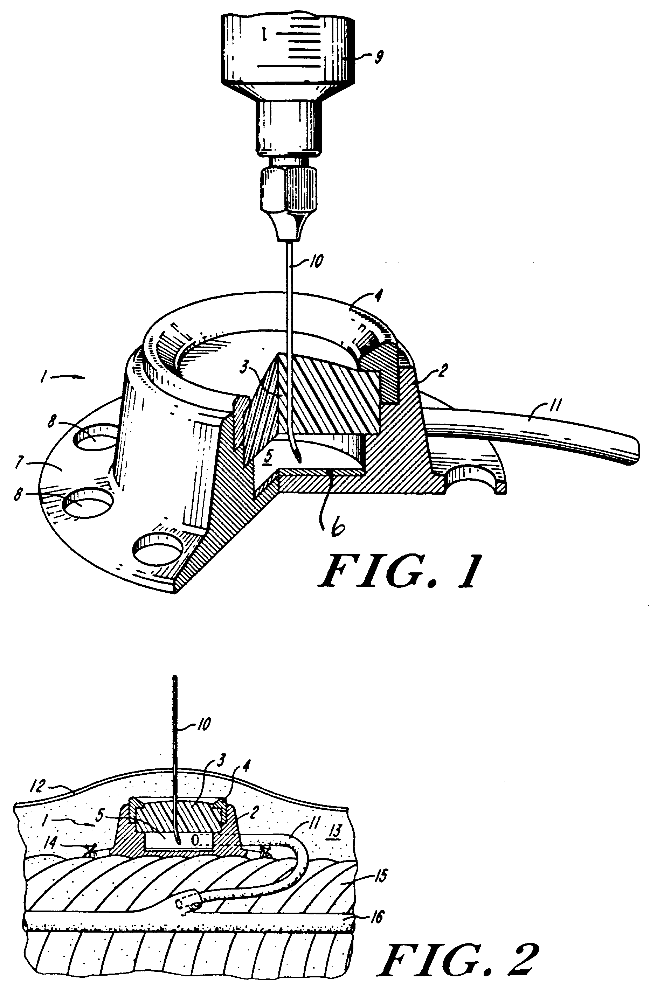

Figures 1 and 2 show a cutaway pictorial view of an implantable assembly according to the present invention. Device 1 of the assembly includes a body portion or housing 2 defining a generally cup-shaped recess, chamber or cavity forming a reservoir 5 for holding treatment fluids or medicine. Housing 2 has an open face which is closed off by a septum or cover 3 held down by a retaining ring 4. Cover 3 is formed of a self-resealing polymer, which is preferably an elastomer such as a silicone rubber or latex, and is adapted to permit access using a hypodermic needle to the reservoir 5 formed by the cover 3 and the housing 2. Housing 2 is formed of a biocompatible material, such as electropolished 316L stainless steel, or other surgical grade steel or biocompatible hard material. At the base of the reservoir 5 a protection plate 6 formed of a suitable material, such as a high durometer silicone rubber, is placed to prevent damage to a needle tip. Housing 2 has a generally cup-shaped form, rising from a base plate 7. Plate 7 has apertures 8 therein evenly spaced about the perimeter of the housing for suturing the device 1 to a layer of tissue when implanting. Also shown in Figure 1 is a syringe 9 having a non-coring needle 10. Syringe 9 is shown by way of illustration having penetrated the septum or cover 3, in position to refill the reservoir. A catheter 11 leads from the reservoir to the vascular system of the subject.

Figure 2 shows a schematic section of the device 1 implanted beneath the skin of a mammal. As shown, device 1 lies beneath the skin 12 in the sub-cutaneous tissue 13 of the mammal. One or more sutures 14 through apertures 8 of the base plate 7 attach device 1 to the muscle fascia 15, and catheter 11 leads from the housing to the vascular system 16 of the mammal. The housing 2 of device 1 has a low profile and a broad flat base. This geometry orients the septum 3 to face outwardly toward the skin, so that the reservoir may be repeatedly and conveniently refilled. Needle 10 is shown in position to refill the reservoir.

It will be appreciated that because device 1 is sutured directly to the mammal, a high degree of manoeuverability of the device or accessibility of the suture apertures 8 is desired for the surgical process of implantation. However, because device 1 connects directly via catheter 11 to the vascular system, the integrity of the catheter connection must also be assured. Furthermore, it is desirable to size the length of the catheter after one end has been placed into a vein and the catheter threaded into position. According to the present invention these ends are achieved by providing in the housing a low profile locking means 20 adapted to receive a separate twist-lockable catheter connector 17 which fits over the sized catheter. Safety means secures the twist-locked catheter to the housing in fixed orientation.

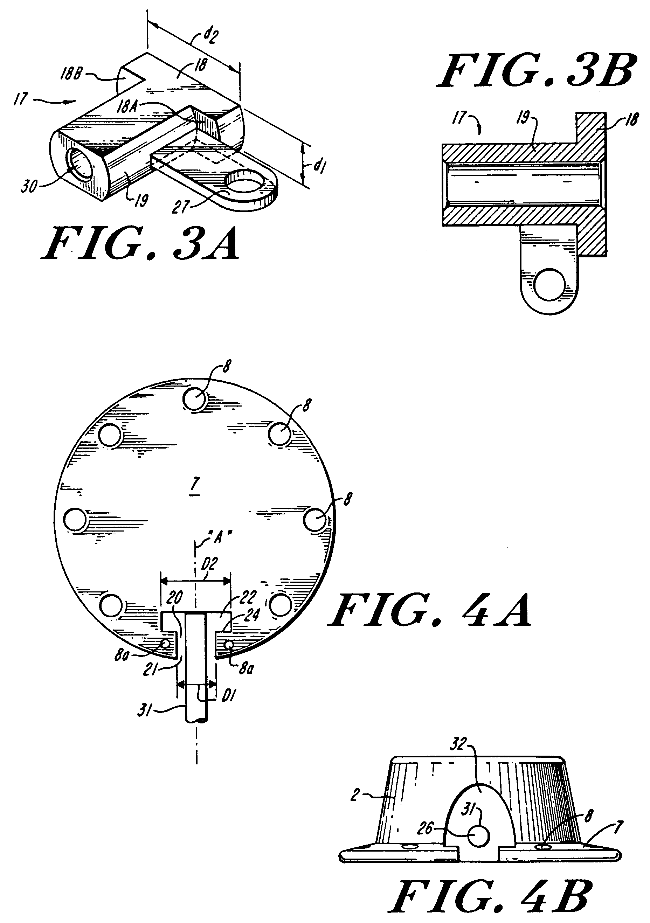

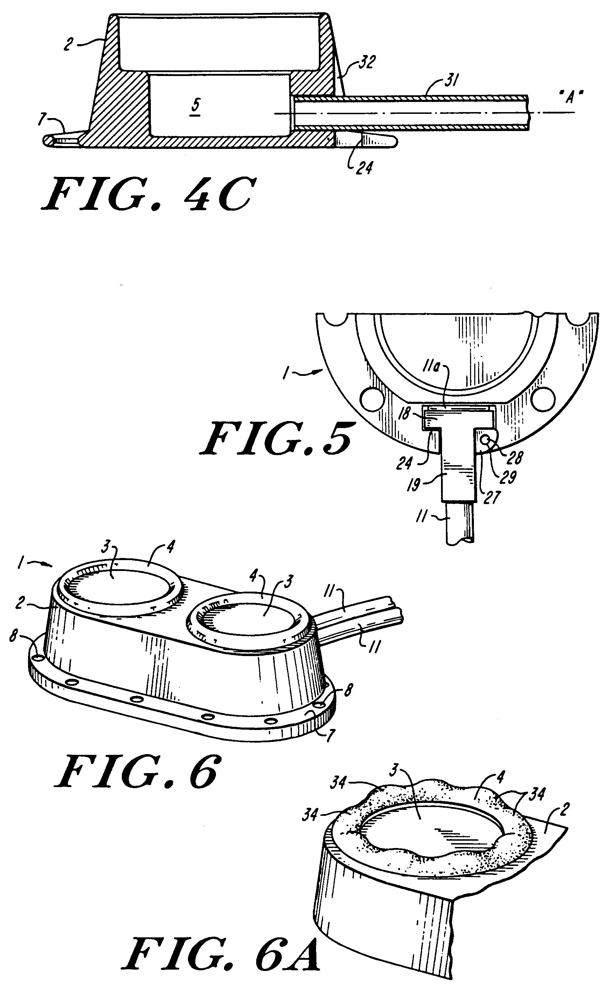

Figures 3A, 3B show views of a catheter end twist-lock connector. Figures 4A, 4B and 4C show corresponding mating locking portion 20 on the housing 2 of device 1, adapted to receive the catheter connector 17 shown in Figures 3A, 3B in accordance with the invention.

Figure 3A is a perspective view from the catheter end of flanged catheter connector 17. Connector 17 comprises a peripheral flange 18 and a central portion or tubular body 19 having a bore 30 therethrough for fitting over a catheter in such a manner as to be slidably moved therealong. Flange 18 comprises opposed arcuate flange segments 18A, 18B extending radially outward from central portion 19. Flange 18 has a cross-dimension d₁ in a first direction and a cross-dimension d₂ in a second direction angularly offset therefrom. Connector 17 also includes a tab 27, discussed further below in relation to Figure 5.

Figure 3B shows a section through the connector of Figure 3A. As shown, flange segments 18A, 18B extend in a direction radially outward from the central flow axis of the catheter. Inwardly thereof, central portion defines a generally tubular body 19 oriented along the catheter flow axis. The tubular body 19 serves as a collar, and has an inner diameter calculated to elastically compress the catheter when the catheter has been slipped over a cannula to attach it to the reservoir.

Figure 4A shows a bottom view of housing 2 of the device 1 for receiving a catheter 11 and connector 17. As shown, the housing comprises the substantially disc-shaped base plate 7 having apertures 8 evenly spaced about the perimeter thereof. A vertical T slot serving as locking portion 20 is formed in base plate 7 with the axis "A" of the T oriented along a radius of the disc. Slot 20 comprises an axial portion serving as input region 21 and an interior region or cross portion 22. As shown, axial portion 21 is aligned with a radius of the base plate 7 and cross portion 22 is perpendicular thereto. The width D₁ of axial portion 21 is slightly greater than the width d₁ in the narrow direction of flange 18 of the catheter connector 17 (Figure 3A) to which it is matched, and less than d₂. Thus the catheter connector 17 may be slidably fitted into the T slot along axis A. Cross portion 22 has a width greater than axial portion 21, allowing rotation of the connector. Thus, when the catheter connector 17 is moved axially into axial portion 21 and butted up against the housing 2, rotation of the catheter connector 17 within cross portion 22 results in positioning the flange 18 behind an inner or confronting surface 24 of cross portion 22. Surface 24 thus traps the catheter connector 17 within the T-slot 20 in the manner of a bayonet mount to prevent axial motion thereof. Figure 4A also shows cannula 31, which is press fit into the housing 2 so as to provide a fluid outlet from the reservoir. Cannula 31 in a prototype embodiment is a straight tube, having a diameter small enough so that the catheter may be slidably fitted thereover, yet large enough to firmly compress the catheter when the catheter connector 17 is placed over the catheter.

Figure 4B shows a side view along the central axis A of the T slot 20 and housing 2. Housing 2 has an outlet aperture or exit port 26 formed along the axis A of slot 20, and communicating directly to the reservoir 5. Cannula 31 is fitted by an interference fit in aperture 26 for receiving the catheter prior to sliding the catheter connector 17 along the T-slot. A rotation of the catheter connector 17 then engages its flange segment 18A, 18B behind an extending arm of which a surface (24 of Figure 4A) thereby engages the catheter connector firmly to prevent its pulling away from the housing. This establishes secure fluid communication between the reservoir and the catheter.

Figure 4C shows a vertical section along the outflow axis "A" of the device 1. A face 32 of housing 2 lying perpendicular to axis A is preferably milled flat when forming the T-slot. The cup-shaped central recess or chamber of the housing is a stepped bore, which defines the reservoir 5 and a circular recess for holding the elastomeric cover or septum (not shown). The cannula 31 extends from the reservoir to receive a catheter.

Figure 5 shows a top view of device 1 with the twist-lock catheter connector 17 mounted in position. As shown, the catheter connector has been inserted in the T-slot and rotated so as to bring flange 18 into engagement behind inner surface 24 of the cross portion of the T. In this position, tab 27 protruding from connector 17 is brought down flush against the base plate 7. Tab 27 is shown held by a structure which provides positive resistance to counter-rotation of the catheter connector 17 which might lead to its detachment. The tab 27 lies in a plane oriented, with respect to the flange 18 of the connector 17 so as to assure that, when held in position against base plate 7, flange 18 is held fully rotated in slot 20, whereby the connector is positively locked in the housing. In the preferred embodiment tab 27 has an aperture 28 therethrough, and may be sutured down against the base plate 7 with sutures 29. The sutures 29 pass through corresponding holes in the base plate 7, shown as holes 8a in Figure 4A. Catheter 11 is compressed between the inner face of the central portion or tubular body 19 of the connector, and the cannula 31 (Figures 4A-4C). In addition, when the connector 17 is installed, the catheter "bunches up" at 11a into a thickened donut-shaped mass which provides an axially directed elastic force along axis A. The resultant pressure between flange 18 and surface 24 further inhibits rotation of connector 17.

Figure 6 shows a perspective view of an implantable assembly having two reservoirs, with the numbered elements corresponding to the identically numbered parts of Figures 1 to 5.

Figure 6A shows a detail of a preferred embodiment of a multi-reservoir implantable assembly, such as the assembly of Figure 6, but having formed thereon bumps or other tactile features which may be felt through the skin when implanted, to identify a reservoir thereof. Retaining ring 4 has a scalloped upper surface with such bumps 34 thereon, so that the bumps 34 outline the cover or septum 3 in a manner easily felt from outside the body. Thus the bumps indicate the location of a reservoir or injection site. In the event it is desired to have more than two reservoirs, which may be placed in a cloverleaf or other cluster configuration, the tactile features preferably include different arrangements of tactile features which additionally each serve as a "code" for the particular medicine which is to be held by that reservoir.

Furthermore the tactile features for distinguishing may take various forms, from a single bump to a coded pattern, and may be placed upon the cover septum itself, or other palpable portion of the body portion.

## Claims

1. An implantable assembly comprising a device (1) having a body portion (2) formed of biocompatible material and including a chamber with an open face, a cover (3) closing the chamber to form a closed reservoir (5) for holding treatment fluid, the body portion (2) having an outlet aperture (26) in fluid communication with the reservoir and a cannula (31) extending therefrom, and further comprising an elastically compressible catheter (11) having a central passage, and collar means to secure the catheter (11) to the cannula (31), characterised by a bayonet type coupling including a locking portion (20) on the device (1) adjacent to the outlet aperture (26) and a separate twist-lockable flanged catheter connector (17) for detachably coupling the catheter (11) and the device (1), the flanged connector having a generally tubular body (19) serving as the collar means, with radially extending flange segments (18A, 18B) and having a central bore (30), the body (19) being slidable over the catheter (11) and its bore (30) having a diameter calculated to elastically compress the catheter (11) between the bore (30) and the outer surface of the cannula, and the locking portion (20) having an input region (21) large enough to permit passage of the flange segments (18A, 18B) and an interior region (22) to accommodate the flange segments (18A, 18B) between a surface (32) of the device (1) and a confronting surface (24) for releasingly engaging the flange segments (18A, 18B) when an end of the catheter (11) is positioned over the cannula (31) and the tubular body (19) is slidingly positioned over the end of the catheter (11) and is then manipulated by a partial revolution for twist locking engagement with the locking portion (20), thereby securing the catheter (11) compressively engaged with the cannula (31).

2. An assembly as claimed in claim 1, characterised in that the body portion (2) defines plural chambers, each with an open face, and wherein the cover means (3) closes each chamber, thereby defining plural closed reservoirs (5), the body portion including an access aperture (26) and a corresponding locking portion (20) for each said reservoir.

3. An assembly as claimed in claim 1, characterised in that the body portion (2) includes a base plate (7) adapted for suturing to the body tissue of the mammal.

4. An assembly as claimed in claim 3, characterised in that the body portion (2) includes means (8a) for securing the connector (17) against rotational movement.

5. An assembly as claimed in claim 1, wherein the catheter (11) has a central passage and an entry end to be coupled to the cannula (31) which defines a flow path to said central passage along a central axis, the bayonet type coupling means detachably coupling said catheter (11) and cannula (31) to establish a continuous flow path between said reservoir (5) and said central passage, wherein said coupling means includes the twist-lockable connector (17) surrounding said catheter for mating with the locking portion (20), the flange segments (18A, 18B) of the connector (17) having a maximum dimension d₁ in a first direction and a maximum dimension d₂ in a second direction rotationally offset from said first direction about the central axis, d₂ being greater than d₁, and said body (19) of the connector (17) having outer dimensions essentially no larger than d₁ and d₂ in said first and second directions, said central axis being perpendicular to said first direction and said second direction.

6. An assembly as claimed in claim 1, comprising plural distinct reservoirs (5) for holding treatment fluid, each having a self-resealing cover (3), and further including tactile means (34) palpable from outside the body and proximal to a particular said reservoir (5), for distinguishing the particular reservoir.

7. An assembly as claimed in claim 6, characterised in that the tactile means (34) defines an outline, palpable through the skin, of the self-resealing cover (3) of the particular reservoir.

8. An assembly as claimed in claim 6 or claim 7, characterised in that the tactile means (34) comprises at least one palpable patterned shape located such that the location of the at least one shape indicates the location of an injection site for the particular reservoir (5), and patterned such that the pattern represents a particular drug to be injected into the particular reservoir.

9. An assembly as claimed in claim 1, wherein said locking portion (20) forms a T-slot into which the catheter (11) and connector (17) slide along a central axis to establish fluid communication with the cannula (31), the connector (17) being rotatable to bring the flange segments (18A, 18B) of the connector into juxtaposition with the confronting surface (24) of the T-slot, thereby preventing disconnecting axial motion of the connector.

## Patentansprüche

1. Implantierbare Zusammenstellung umfassend eine Vorrichtung (1) mit einem aus biokompatiblem Material gebildeten Körperabschnitt (2), die eine Kammer mit offener Vorderseite enthält, einen Deckel (3), der die Kammer zur Bildung eines geschlossenen Speichers (5) abschließt, der eine Behandlungsflüssigkeit enthält, wobei der Körperabschnitt (2) eine Austrittsöffnung (26) in Fließverbindung mit dem Speicher und eine sich daraus erstreckende Kanüle (31) aufweist, weiterhin umfassend einen elastisch zusammendrückbaren Katheter (11) mit einem zentralen Durchgang, und einer kragenförmigen Einfassung zur Befestigung des Katheters (11) an die Kanüle (31), gekennzeichnet durch eine bajonettartige Verkopplung umfassend ein an die Austrittsöffnung (26) angrenzendes Feststellteil (20) an der Vorrichtung (1), und ein getrenntes, durch Drehung arretierbares Flanschkatheterverbindungsstück (17) zur demontierbaren Verknüpfung des Katheters (11) mit der Vorrichtung (1), wobei das Flanschverbindungsstück einen im allgemeinen röhrenförmigen Körper (19) aufweist, der als die kragenförmige Einfassung dient, mit radial sich erstreckenden Flanschsegmenten (18A, 18B) und einer zentralen Bohrung (30), wobei der Körper (19) über den Katheter (11) schiebbar ist und dessen Bohrung (30) einen Durchmesser aufweist, der so berechnet ist, daß der Katheter (11) zwischen der Bohrung (30) und der äußeren Oberfläche der Kanüle elastisch zusammengedrückt wird, und das Feststellteil (20) einen Eingangsbereich (21) aufweist, der groß genug ist, um den Durchlaß der Flanschsegmente (18A, 18B) zu gestatten, und einen inneren Bereich (22) zur Aufnahme der Flanschsegmente (18A, 18B) zwischen einer Oberfläche (32) der Vorrichtung (1) und einer gegenüberliegenden Oberfläche (24) zum lösbaren Eingreifen der Flanschsegmente (18A, 18B), wenn ein Ende des Katheters (11) über die Kanüle (31) positioniert wird, und der röhrenförmige Körper (19) über das Ende des Katheters (11) schiebbar positioniert wird und dann durch eine teilweise Drehung betätigt wird zum durch Drehung zu arretierenden Eingriff mit dem Feststellteil (20), wodurch der durch Druck mit der Kanüle (31) eingegriffene Katheter (11) befestigt wird.

2. Zusammenstellung nach Anspruch 1, dadurch gekennzeichnet, daß durch den Körperabschnitt (2) mehrfache Kammern mit jeweils offener Vorderseite definiert werden, und worin die Deckeleinrichtung (3) jede Kammer abschließt, wodurch mehrfache geschlossene Speicher (5) definiert werden, wobei der Körperabschnitt eine Zutrittsöffnung (26) und ein entsprechendes Feststellteil (20) für jeden besagten Speicher umfaßt.

3. Zusammenstellung nach Anspruch 1, dadurch gekennzeichnet, daß der Körperabschnitt (2) eine Bodenplatte (7) umfaßt, die zum Nähen an das Körpergewebe des Mammals angepaßt ist.

4. Zusammenstellung nach Anspruch 3, dadurch gekennzeichnet, daß der Körperabschnitt (2) eine Einrichtung (8a) enthält, um das Verbindungsstück (17) gegen Drehbewegung zu sichern.

5. Zusammenstellung nach Anspruch 1, worin der Katheter (11) einen zentralen Durchgang und ein Eintrittsende aufweist, das an die Kanüle (31) gekoppelt wird, die einen Strömungsweg zu besagtem zentralen Durchgang entlang einer Zentralachse definiert, wobei die bajonettartige Verkopplungseinrichtung den besagten Katheter (11) und die Kanüle (31) demontierbar verbindet zur Bildung eines kontinuierlichen Strömungsweges zwischen besagtem Speicher (5) und besagtem zentralem Durchgang, worin die besagte Verkopplungseinrichtung das durch Drehung arretierbare Verbindungsstück (17) enthält, das den besagten Katheter zum Eingreifen in das Feststellteil (20) umgibt, wobei die Flanschsegmente (18A, 18B) des Verbindungsstücks (17) eine maximale Abmessung d₁ in einer ersten Richtung und eine maximale Abmessung d₂ in einer zweiten Richtung aufweisen, die von besagter erster Richtung durch Drehung um die Zentralachse versetzt ist, wobei d₂ größer als d₁ ist, und besagter Körper (19) des Verbindungsstücks (17) äußere Abmessungen aufweist, die im wesentlichen nicht größer als d₁ und d₂ in besagter erster und zweiter Richtung sind, wobei die besagte Zentralachse zu besagter erster Richtung und besagter zweiter Richtung senkrecht steht.

6. Zusammenstellung nach Anspruch 1, die mehrfache getrennte Speicher (5) umfaßt, die Behandlungsflüssigkeit enthalten, wobei jeder einen selbstwiederabdichtenden Deckel (3) aufweist, und weiterhin umfassend Tasteinrichtungen (34), die von außerhalb des Körpers tastbar sind und proximal zu einem speziellen besagten Speicher (5) liegen, zur Unterscheidung des speziellen Speichers.

7. Zusammenstellung nach Anspruch 6, dadurch gekennzeichnet, daß durch die Tasteinrichtungen (34) durch die Haut tastbare Konturen des selbstwiederabdichtenden Deckels (3) des speziellen Speichers definiert werden.

8. Zusammenstellung nach Anspruch 6 oder Anspruch 7, dadurch gekennzeichnet, daß die Tasteinrichtungen (34) mindestens ein tastbares Musterprofil umfassen, das derart angeordnet ist, daß die Lage von mindestens einem Profil den Ort einer Injektionsstelle für den speziellen Speicher (5) anzeigt, und derart geformt ist, daß das Muster ein bestimmtes, in den speziellen Speicher zu injizierendes Arzneimittel darstellt.

9. Zusammenstellung nach Anspruch 1, worin besagtes Feststellteil (20) eine T-Nut bildet, in die der Katheter (11) und das Verbindungsstück (17) entlang einer Zentralachse zur Bildung einer Fließverbindung mit der Kanüle (31) gleiten, wobei das Verbindungsstück (17) drehbar ist, um die Flanschsegmente (18A, 18B) des Verbindungsstücks mit der gegenüberliegenden Oberfläche (24) der T-Nut in Nebeneinanderstellung zu bringen, wodurch eine entkoppelnde Bewegung des Verbindungsstücks in Achsrichtung verhindert wird.

## Revendications

1. Ensemble implantable comportant un dispositif (1) ayant une partie de corps (2) réalisée en matériau bio-compatible et comportant une chambre ayant une face ouverte, un couvercle (3) fermant la chambre pour former un réservoir fermé (5) destiné à contenir un fluide de traitement, la partie de corps (2) ayant une ouverture de sortie (26) en communication de fluide avec le réservoir et une canule (31) s'étendent à partir de celle-ci, et comportant en outre un cathéter élastiquement compressible (11) ayant un passage central, et des moyens forment collier pour fixer le cathéter (11) sur la canule (31), caractérisé en ce qu'il comporte un accouplement du type à balonnette comportant une partie de verrouillage (20) située sur le dispositif (1), adjacente à l'ouverture de sortie (26), et un raccord de cathéter séparé (17), muni d'une collerette, verrouillable en rotation pour coupler le cathéter (11) et le dispositif (1), le raccord muni d'une collerette ayant un corps (19) généralement tubulaire servant de moyens formant collier, des segments de collerette (18A, 18B) s'étendant radialement et ayant un alésage central (30), le corps (19) pouvant coulisser sur le cathéter (11) et son alésage (30) ayant un diamètre calculé pour comprimer élastiquement le cathéter (11) entre l'alésage (30) et la surface extérieure de la canule, et la partie de verrouillage (20) ayant une zone d'entrée (21) assez large pour permettre le passage des segments de collerette (18A, 18B) et une zone intérieure (22) pour recevoir les segments de collerette (18A, 18B) entre une surface (32) du dispositif (1) et une surface en vis-à-vis (24) destinée à venir en contact, de manière libérable, avec les segments de collerette (18A, 18B) quand une extrémité du cathéter (11) est positionnée sur la canule (31) et le corps tubulaire est positionné de manière coulissante sur l'extrémité du cathéter (11) et ensuite est manipulé par mise en rotation partielle pour venir en contact de verrouillage en rotation avec la partie de verrouillage (20), de manière à immobiliser le cathéter (11) en prise de manière comprimée avec la canule (31).

2. Ensemble selon la revendication 1, caractérisé ce que la partie de corps (2) définit plusieurs chambres, chacune ayant une face ouverte, et dans lequel les moyens formant couvercle (3) ferment chaque chambre, de manière à définir plusieurs réservoirs fermés (5), la partie de corps ayant une ouverture d'accès (26) et une partie correspondante de verrouillage (20) pour chacun desdits réservoirs.

3. Ensemble selon la revendication 1, caractérisé en ce que la partie de corps (2) comporte une plaque de base (7) adaptée pour être suturée aux tissus corporels du mammifère.

4. Ensemble selon la revendication 3, caractérisé en ce que la partie de corps (2) comporte des moyens (8a) pour immobiliser le raccord (17) à l'encontre d'un mouvement de rotation.

5. Ensemble selon la revendication 1, dans lequel le cathéter (11) a un passage central et une extrémité d'entrée destinée à être couplée à la canule (31) qui définit un trajet d'écoulement vers le passage central le long d'un axe central, des moyens de couplage du type à baïonnette couplant de manière amovible ledit cathéter (11) et la canule (31) pour établir un trajet d'écoulement continu entre ledit réservoir (5) et ledit passage central, dans lequel lesdits moyens de couplage comportent un raccord verrouillable en rotation (17) entourant ledit cathéter pour l'associer à la partie de verrouillage (20), les segments de collerette (18A, 18B) du raccord (17) ayant une dimension maximale d1 dans une première direction et une dimension maximale d2 dans une seconde direction décalée en rotation par rapport à ladite première direction autour de l'axe central, d2 étant plus grand que d1, et ledit corps (19) du raccord (17) ayant des dimensions extérieures pratiquement pas plus grandes que d1 et d2 dans lesdites première et deuxième directions, ledit axe central étant perpendiculaire à ladite première direction et à ladite seconde direction.

6. Ensemble selon la revendication 1, comportant plusieurs réservoirs distincts (5) pour contenir du fluide de traitement, chacun ayant un couvercle auto-obturateur (3), et comportant de plus des moyens tactiles (34) palpables de l'extérieur du corps et situés à proximité d'un réservoir particulier (5), afin de distinguer le réservoir particulier.

7. Ensemble selon la revendication 6, caractérisé en ce que les moyens tactiles (34) définissent un contour, palpable à travers la peau, du couvercle auto-obturateur (3) du réservoir particulier.

8. Ensemble selon la revendication 6 ou 7, caractérisé en ce que les moyens tactiles (34) comportent au moins une forme configurée palpable localisée de sorte que l'emplacement de la au moins une forme indique l'emplacement d'un site d'injection pour le réservoir particulier (5), et configurée de façon que la configuration représente un médicament particulier à injecter situé dans un réservoir particulier.

9. Ensemble selon la revendication 1, dans lequel ladite partie de verrouillage (20) forme une fente en T dans laquelle le cathéter (11) et le raccord coulissent le long de l'axe central pour établir une communication de fluide avec la canule (31), le raccord (17) pouvant être mis en rotation pour amener les segments de collerette (18A, 18B) du raccord en juxtaposition avec la surface en vis-à-vis (24) de la fente en T, en empêchant ainsi un mouvement axial de déconnexion du raccord.
